(19) Europäisches Patentamt
European Patent Office
Office européen des brevets

(11) **EP 4 350 711 A1**

(12) **EUROPEAN PATENT APPLICATION**
published in accordance with Art. 153(4) EPC

(43) Date of publication:
**10.04.2024 Bulletin 2024/15**

(51) International Patent Classification (IPC):
**G16H 50/50** (2018.01)

(21) Application number: **22940510.5**

(22) Date of filing: **19.08.2022**

(86) International application number:
**PCT/JP2022/032302**

(87) International publication number:
**WO 2024/038618 (22.02.2024 Gazette 2024/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **UT-Heart Inc.**
**Tokyo, 154-0003 (JP)**

(72) Inventors:
• **HISADA, Toshiaki**
  **Tokyo 1540003 (JP)**
• **SUGIURA, Seiryo**
  **Tokyo 1540003 (JP)**
• **WASHIO, Takumi**
  **Tokyo 1540003 (JP)**
• **OKADA, Jun-ichi**
  **Tokyo 1540003 (JP)**
• **FUJIU, Katsuhito**
  **Tokyo 1138654 (JP)**

(74) Representative: **Hasegawa, Kan**
**Patentanwaltskanzlei Hasegawa**
**Untere Hauptstraße 56**
**85354 Freising (DE)**

(54) **METHOD FOR UTILIZING IN-SILICO HEART DISEASE DATABASE, PROGRAM FOR UTILIZING IN-SILICO HEART DISEASE DATABASE, AND INFORMATION PROCESSING DEVICE**

(57) An information processing technology capable of increasing prediction accuracy of causing cardiac diseases, performing appropriate therapy and increasing efficiency of drug development is constructed by utilizing "in-silico cardiac disease database." An electrocardiogram and the like obtained from "in-silico cardiac disease database" which is the database storing a simulation result of a cardiac model of virtual disease where various factor related to cardiac disease are changed are inputted to a classifier for performing an automatic diagnosis of the cardiac disease, and an influencing factor associated with the cardiac disease is identified as a biomarker by comparing distributions of a variation amount of a factor between a positive group and a negative group. In addition, precise diagnosis of an individual is easily performed by identifying the cardiac model having the electrocardiogram and the like appropriate to the actual electrocardiogram and the like of the individual in the in-silico cardiac disease database. Furthermore, the process of drug development is efficiently performed by extracting a group having a predetermined feature from the in-silico cardiac disease database and simply evaluating an effect of an administration of a medical agent.

Fig. 2

EP 4 350 711 A1

**Description**

TECHNICAL FIELD

The present invention relates to an in-silico cardiac disease database utilization method, an in-silico cardiac disease database utilization program and an information processing device.

BACKGROUND ART

Cardiac simulation technologies, which reproduce activities of hearts of healthy people or cardiac disease patients as a physical or physiological mathematical model on a computer, have been put to practical use in recent years. When the cardiac simulation technologies are used, it is possible to mathematically reproduce a cardiac model of an individual patient on a computer and then virtually perform a simulation of the cardiac model on the computer and predict the condition of the heart after the treatment. Consequently, a doctor can support the individual patient to select an optimum therapeutic method.
For example, in accordance with Patent Document 1 and Non-Patent Document 1, before performing a surgical operation of the heart, the cardiac model reproducing a state before the operation is created first and then the cardiac simulation is performed by applying a plurality types of available operations to the cardiac model on the computer. Thus, it is possible to compare the hemodynamic status after the treatment and the load on the heart to select better type of operation. As another example, in accordance with Patent Document 2 and Non-Patent Document 2, when cardiac resynchronization therapy (CRT) is applied to a predetermined patient by a biventricular pacing, whether or not the positions of the electrodes for transmitting an electrical stimulation outputted from a CRT device to the heart are appropriate and the degree of the effect can be quantitatively confirmed for each patient before the operation by advance simulation. The above described examples are regarded as tailor made medical treatments based on the cardiac simulation technologies.

On the other hand, different from the idea of performing the tailor made medical treatments by regenerating the heart of individual patient, based on both the cardiac simulation technologies and the massively parallel computation achieved by recent super computers, it is possible to create "in-silico cardiac disease database" which is the database formed by creating a large number of virtual cardiac models of the cardiac diseases on the computer where various macro and micro factors associated with the cardiac diseases are variously changed from the normal value with respect to a normal and standard cardiac simulation model and storing the result of the cardiac simulation. Actually, more than 17 thousand of cardiac models are created for performing the simulation in Non-Patent Document 3. In the in-silico cardiac disease database, the standard 12-lead electrocardiograms and the data corresponding to various parameters of the echocardiography can be easily obtained from the result of the cardiac simulation by incidental calculation. Thus, the in-silico cardiac disease database can be used not only for the fundamental medical research but also for the clinical medical research. Note that the term of "in-silico cardiac disease database" is used instead of the term of "cardiac disease database" for distinguishing the in-silico cardiac disease database from the cardiac disease database which is based on an actual clinical data in the hospital.

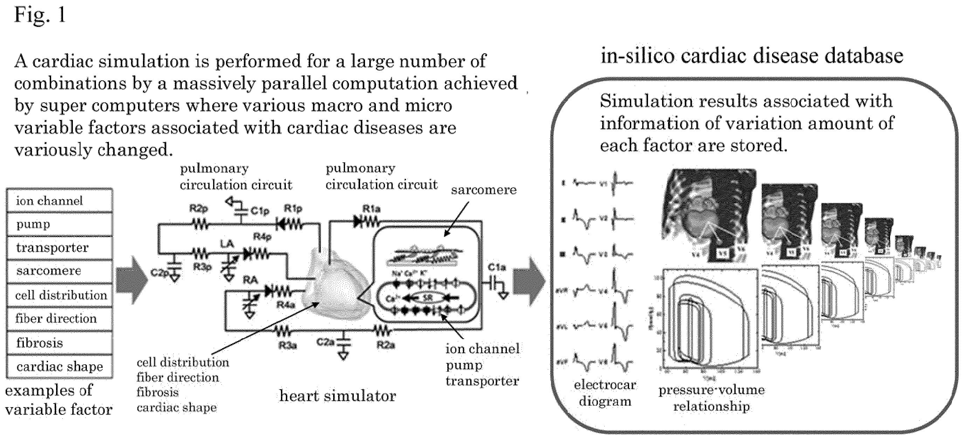

Fig. 1 shows the concept of the in-silico cardiac disease database.

PRIOR ART DOCUMENTS

[Patent Documents]

[Patent document 1] Japanese Patent No. 6300244
[Patent document 2] Japanese Unexamined Patent Application Publication No. 2017-033227
[Patent document 3] Japanese Unexamined Patent Application Publication No. 2022-040892

[Non-Patent Documents]

[Non-Patent Document 1] Kariya T, Washio T, Okada J, Nakagawa M, Watanabe M, Kadooka Y, Sano S, Nagai R, Sugiura S, Hisada T. Personalized Perioperative Multi-scale, Multi-physics Heart Simulation of Double Outlet Right Ventricle. Annals of Biomedical Engineering. 2020. DOI: 10. 1007/s10439-020-02488-y
[Non-Patent Document 2] Okada J, Washio T, Nakagawa M, Watanabe M, Kadooka Y, Kariya T, Yamashita H, Yamada Y, Momomura S, Nagai R, Hisada T, Sugiura S. Multi-scale, tailor-made heart simulation can predict the effect of cardiac resynchronization therapy. Journal of Molecular and Cellular Cardiology. 2017; 108: 17-23.
[Non-Patent Document 3] Research Organization for Information Science and Technology, high-performance general purpose computer high level utilization project in 2020, Fugaku Achievement Acceleration Program "Overcoming heart failure pandemic with innovative integration of multi-scale heart simulator and large-scale clinical data" result report f202_r2.pdf (rist.or.jp)
[Non-Patent Document 4] Mason J. W., Straus D. G, Vaglio M., Badilini F., Correction of the QRS duration for the heart rate. I Electrocardiol.1-4, 2019.
[Non-Patent Document 5] H. Iwasa, T. Itoh, R. Nagai, Y. Nakamura, T. Tanaka, Twenty single nucleotide polymorphisms (SNPs) and their allelic frequencies in four genes that are responsible for familial long QT syndrome in the Japanese population, Journal of Human Genetics, 45, 182-183, 2000.
[Non-Patent Document 6] Okada J, Yoshinaga T, Kurokawa J, Washio T, Furukawa T, Sawada K, Sugiura S, Hisada T. Screening SYSTEM for drug-induced arrhythmogenic risk combining a patch clamp and heart simulator. Science Advances. 1 (4). 2015.

## DISCLOSURE OF THE INVENTION

[Problems to be Solved by the Invention]

It is said that the number of the patients of heart failure, which is the disease that causes the deterioration of the quality of life and causes death due to the disorder of the heart caused by various reasons such as myocardial infarction, cardiomyopathy and valve disease, is 1.2 million in Japan. This number is larger than the number of the patients of cancer since it is said that the number of the patients of cancer is 1 million. It is estimated that the number of the patients of atrial fibrillation, which is a kind of arrhythmia is approximately 0.8 million only by counting the number found in the medical examination. It is said that the number of the patients of atrial fibrillation exceeds 1 million if the number of potential patients is included.

From the economic viewpoint, medical cost related to the circulatory diseases excluding cerebrovascular disease reaches 4.1 trillion yen in Japan. This cost is almost same as 4.2 trillion yen which is the medical cost of cancer. Accordingly, it is an urgent issue to certainly and early detect the cardiac diseases and develop a reasonable therapeutic method. From the viewpoint of early detecting the cardiac diseases, an electrocardiogram and an echocardiography are frequently used since they are easy inspection means. In particular, as for the electrocardiogram, automatic diagnosis is put to practical use by using a software installed in a device, an external software or the like. Furthermore, automatic diagnosis using AI which learns a large amount of teacher data by machine learning has been eagerly developed recently. Although AI is relatively easy, a certain practicality can be expected. However, AI is basically the method for automatically finding the regularity and the relativeness from a large amount of data. It is pointed out about AI that there is "black-box problem" which is the problem that the basis of the derived answer is unclear. Therefore, the research of Explainable AI has been done recently. However, the most fundamental limitation of AI is that AI merely performs prediction and explanation superficially on the given data. The same can be said about the automatic diagnosis without using AI. Accordingly, in order to remarkably improve prediction accuracy and achieve essential treatment, it is required to introduce a new method going further into the truth behind the superficial prediction and explanation on the data of the electrocardiogram and the echocardiography. Namely, the method going further into the physiological mechanism of the cardiac diseases is required.

Based on the above described viewpoints, the present invention aims for constructing an information processing technology capable of certainly and early detecting cardiac diseases and developing a reasonable therapeutic method utilizing the above described "in-silico cardiac disease database."

[Means for Solving the Problem]

As the first proposal, an in-silico cardiac disease database, which is the database formed by creating a large number of cardiac models on the computer where various macro and micro factors associated with the cardiac diseases are variously changed from the normal value with respect to a normal and standard cardiac simulation model and storing the result of the cardiac simulation and the electrocardiogram and echocardiographic parameter obtained by incidental calculation, is utilized as follows. Namely, the electrocardiogram and the echocardiographic parameter of each of the cardiac models in the in-silico cardiac disease database are inputted to an automatic diagnostic tool for performing the diagnosis. Thus, the cardiac models are classified into two groups: one is the group of positive for cardiac diseases and the other is the group of negative for cardiac diseases. Note that the group of positive can be further classified into a plurality of groups depending on the degree of the progress of the cardiac diseases. In addition, the automatic diagnostic tool can be AI which learns a large number of actual electrocardiograms and actual echocardiographic parameters as the teacher data by machine learning or a software without depending on AI accompanying a diagnostic tool, for example.

Note that the explanation "electrocardiogram and echocardiographic parameter" can be only one of the electrocardiogram and the echocardiographic parameter without being limited to both of them. The same can be said in the following explanation.

As the second proposal, the cardiac model in the in-silico cardiac disease database having the electrocardiogram and the echocardiographic parameter most approximate (similar) to the actual electrocardiogram and the actual echocardiographic parameter of a predetermined individual is identified. However, as described later, the electrocardiogram is especially modified in various ways. Thus, it is required to appropriately convert the electrocardiogram in the in-silico cardiac disease database and the actual electrocardiogram of the individual before determining the similarity. AI or the other mathematical methods can be used for determining the similarity.

As the third proposal, the group having a predetermined feature is intentionally extracted from the in-silico cardiac disease database as a subset focusing on the simulation result or the combination of the variation amount of the one or more factors. For example, the group of hearts having a high risk of the arrhythmia can be obtained by extracting the simulation results having a long QT interval in the electrocardiogram. As an example of focusing on the variation amount of the factor, the group of hearts having the trait distribution of a predetermined actual group (e.g., Japanese) can be obtained by extracting the simulation results so that the variation amount of each factor becomes a specified distribution. Then, the state of changing the electrocardiogram and the echocardiographic parameter is statistically evaluated assuming a case where a medical agent is administered to the group of hearts obtained as described above. Here, it is assumed that the quantitative data is experimentally given how the medical agent changes each factor. However, the specific method will be explained in the example of the third embodiment.

[Effects of the Invention]

According to the first proposal, an influencing factor or influencing factors associated with the cardiac disease can be identified by analyzing the distribution of the variation amount of each factor for each group of the cardiac models in the in-silico cardiac disease database classified into positive and negative by the diagnosis. Thus, it is possible to develop the technology of predicting the onset of the cardiac disease with high accuracy using the influencing factor or the influencing factors as a physiological biomarker. Furthermore, a pathogenic mechanism of causing the cardiac disease is predicted based on a physiological causal relationship between the influencing factor or the influencing factors. Thus, it is possible to provide the information for developing the therapeutic method capable of suppressing a variation of the influencing factor or the influencing factors.

According to the second proposal, the cardiac model having the electrocardiogram and the echocardiographic parameter most approximate to the actual electrocardiogram and the actual echocardiographic parameter of a predetermined individual is identified in the in-silico cardiac disease database. Since the variation amount of each factor forming the heart is realized, it is considered that the cardiac model indicates the physiological state of the individual. In particular, the influencing factor or the influencing factors associated with various cardiac diseases are identified as a biomarker and the distributions of the variation amount of the factors are obtained for each of the positive and negative groups in the above described first proposal. Thus, the distribution of the variation amount of the factor and the variation amount of each factor of the individual are compared with each other to precisely diagnose the health condition of the individual in association with the cardiac diseases. As described above, the present invention can be led to the precision medicine only by an easy inspection of the electrocardiogram and the echocardiography.

According to the third proposal, the group (e.g., group having high risk of arrhythmia) of interest to the users of the present invention is intentionally extracted and the effect of administering the medical agent to the group can be easily evaluated on the computer. Thus, this can be a tool for accelerating the drug development. For example, the statistic variation of the electrocardiogram can be easily evaluated in the case where a predetermined medical agent is administrated to the group having the trait of Japanese. Thus, the present invention can be replaced with TQT study (thorough QT/QTc study) performed as a cardiotoxicity screening which is necessary for the drug development. The above described evaluation can be easily performed without performing the cardiac simulation again as specifically explained in the third embodiment.

BRIEF DESCRIPTION OF THE DRAWINGS

Fig. 1 is a drawing showing a concept of in-silico cardiac disease database.
Fig. 2 is a drawing showing a functional configuration of an information processing device 10 for providing information for identifying factors associated with a cardiac disease, analyzing a mechanism and developing a reasonable therapeutic method related to the first embodiment.
Fig. 3 is a flowchart showing an example of an information processing related to the first embodiment.

Fig. 4 is a drawing showing an example of a system configuration and a hardware configuration related to the first embodiment.

Fig. 5 is a drawing showing an outline of constitutional factors of an electrocardiogram and an example of a coverage of in-silico cardiac disease database.

Fig. 6 is a drawing showing a functional configuration of an information processing device 20 for identifying a cardiac model in the in-silico cardiac disease database having an electrocardiogram and echocardiographic parameter most approximate to actual electrocardiogram and actual echocardiographic parameter of an individual related to the second embodiment and performing diagnosis.

Fig. 7 is a flowchart showing an example of procedures of an information processing related to the second embodiment.

Fig. 8 is a drawing showing a functional configuration of an information processing device 30 for easily evaluating an effect of an administration of a medical agent to a group having a predetermined feature related to the third embodiment.

Fig. 9 is a flowchart showing an example of procedures of an information processing related to the third embodiment.

Fig. 10 is a drawing showing a functional configuration of an information processing device 40 for providing an information for identifying factors associated with a therapy effect, analyzing a mechanism and developing a reasonable therapeutic method related to the other embodiments.

Fig. 11 is a flowchart showing an example of procedures of an information processing related to the other embodiment.

MODES FOR CARRYING OUT THE INVENTION

Hereafter, the embodiments of the present invention is explained.
Note that a plurality of embodiments can be combined with each other as long as there is no inconsistency.

[First embodiment]

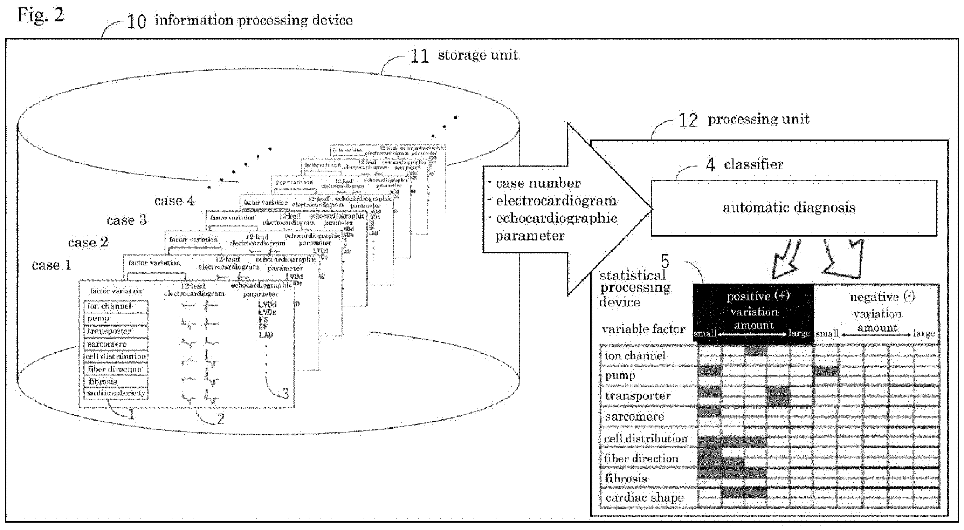

The first embodiment provides the information for identifying factors associated with an onset of a cardiac disease, analyzing a mechanism of the cardiac disease and developing a reasonable therapeutic method. Fig. 2 is a drawing showing an example of the first embodiment. Fig. 2 shows a functional configuration of the information processing device 10 for providing the information for identifying the factors associated with the onset of the cardiac disease, analyzing the mechanism of the cardiac disease and developing the reasonable therapeutic method. The information processing device 10 is composed of a storage unit 11 and a processing unit 12 where an entire or a part of the data of in-silico cardiac disease database is imported in the storage unit 11. As the concept is shown in Fig. 1, a large number of results of the cardiac simulation is stored in the in-silico cardiac disease database. The cardiac simulation is performed by variously changing the factors which may be associated with the cardiac disease from the normal value. A predetermined combination of the variation amount of each factor corresponds to one case. All results of the cardiac simulation are stored in each case together with a variation amount data 1 of each factor. Note that the factors shown in Fig. 2 roughly show examples of the factors. For example, as for ion channels, the segmentalized ion channel such as potassium, sodium and calcium is the factor.

The cardiac simulation is achieved by the simulation related to electrical phenomenon and physical phenomenon. In the electrical simulation, the cardiac model is embedded in a body model (Fig. 1). As a result, the momentarily varied potential is generated on the surface of the body model. An electrocardiogram 2 can be obtained by extracting the potentials at nine electrode positions for the standard 12-lead electrocardiogram on the body and performing a slight calculation. In addition, the momentarily varied movement of the cardiac wall can be obtained from the physical simulation. Thus, various echocardiographic parameters 3 (e.g., the change of the diameter of the left ventricle and the cardiac blood output) can be calculated by processing the coordinates of the cardiac wall. All of them are prepared in the in-silico cardiac disease database. The variation amount data 1, the electrocardiogram 2 and the echocardiographic parameter 3 of each factor of each case is stored in the storage unit 11 at the minimum. The processing unit 12 is composed of a classifier 4 and a statistical processing device 5. The case number, the electrocardiogram and the echocardiographic parameter are inputted to the classifier 4 for each case stored in the storage unit 11. The automatic diagnosis in the classifier 4 can be performed by AI which learns a large number of actual electrocardiograms and actual echocardiographic parameter as the teacher data by machine learning or a software without depending on AI accompanying a diagnostic tool, for example. Each case inputted from the storage unit 11 is diagnosed by the classifier 4 and classified into the groups of a positive (+) and a negative (-) about a predetermined cardiac disease. In Patent document 3, the AI for diagnosing the heart failure by the machine learning (deep learning) of the actual electrocardiogram has been developed.

The statistical processing device 5 is a program statistically organizing the variation amount of each factor associated with each case number since the variation amount can be realized by the statistical processing device 5.

Specifically, in the example of Fig. 2, the variation amount of each factor consists of 5 stages from small to large. For each factor of each case, 1 is added to a relevant part of five-stages of counting boxes in the positive (+) group or the negative (-) group. By performing the above described operation for all cases, the distribution of the positive (+) group and the distribution of the negative (-) group can be obtained for the variation amount of each factor. In the example shown in Fig. 2, the image of the distribution is shown by the difference of density of each of the counting boxes. When the significant difference is not seen between the positive (+) group and the negative (-) group in a predetermined factor, it can be assumed that the predetermined factor is not associated with the cardiac disease to be targeted. On the contrary, when the significant difference is seen between them, it can be assumed that the predetermined factor is associated with the cardiac disease to be targeted. For example, when the distribution of the factor in the positive (+) group is continuously increased, it can be considered that the onset risk of the cardiac disease increases as the variation (difference) from the normal value is larger in a predetermined factor. The above described influencing factor can be identified as a physiological biomarker of the cardiac disease to be targeted. Furthermore, by analyzing the variation distribution between the influencing factors while considering the physiological causal relationship (e.g., the contractile force of the sarcomere is adjusted by the calcium ion concentration), it is possible to provide the information for predicting the mechanism of causing the cardiac disease and developing the therapeutic method capable of reasonably suppressing the variation of the influencing factor or the influencing factors.

In the example shown in Fig. 2, the classifier 4 performs the automatic diagnosis by classifying the cardiac models into two groups of the positive (+) and the negative (-) for a predetermined cardiac disease. However, it is also possible to perform the automatic diagnosis by classifying the factor into a plurality of groups depending on the degree of the progress of the cardiac disease. For example, as for the heart failure, since the classification of the cardiac function of NYHA (New York Heart Association) is frequently used where the cardiac function is classified into four degrees from the first degree (no symptoms in ordinary physical activity although heart disease is seen) to the fourth degree (symptoms of heart failure and anginal pain are seen even at rest), it is possible to use the classifier for classifying the cardiac models based on the classification of the cardiac function of NYHA.

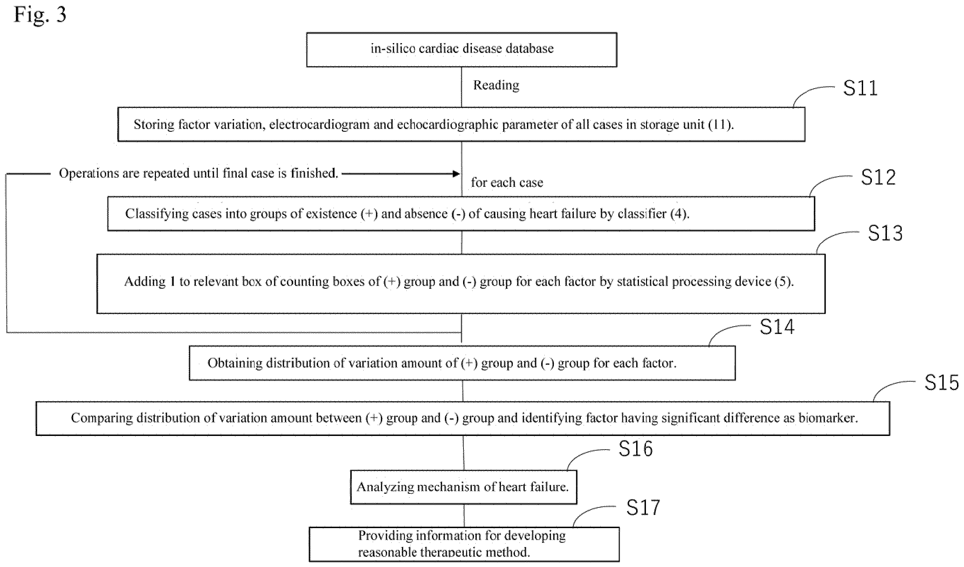

Fig. 3 is a flowchart showing an example of procedures of the above described information processing.

[Step S11] A variation 1 of the factor, an electrocardiogram 2 and an echocardiographic parameter 3 of all cases are read from the in-silico cardiac disease database and stored in the storage unit 11. The case number is assigned to each case.

[Step S12] The electrocardiogram 2 and the echocardiographic parameter 3 of each case are extracted from the storage unit 11 and entered into the classifier 4. Thus, the cases are classified into any one of the group of existence (+) of cardiac disease and the group of absence (-) of cardiac disease.

[Step S13] In the statistical processing device 5, the counting boxes indicating the variation amount of each factor are prepared for each of the (+) group and the (-) group. For each factor, 1 is added to a relevant box of the counting boxes in any one of the (+) group and the (-) group.

Returning to Step S12, similar operations are repeated until the final case is finished.

[Step S14] A distribution of the values of the counting boxes of the (+) group and the values of the counting boxes of the (-) group, which is a distribution of the variation amount, can be obtained for the variation amount of each factor.

[Step S15] The distribution of the variation amount is compared between the (+) group and the (-) group and the factor having a significant difference is identified as a biomarker.

[Step S16] The mechanism of causing the cardiac disease is analyzed considering the causal relationship between the factors.

[Step S17] The information for developing the reasonable therapeutic method is provided from the biomarker identified in Step S15 and the mechanism of causing the cardiac disease analyzed in Step S16.

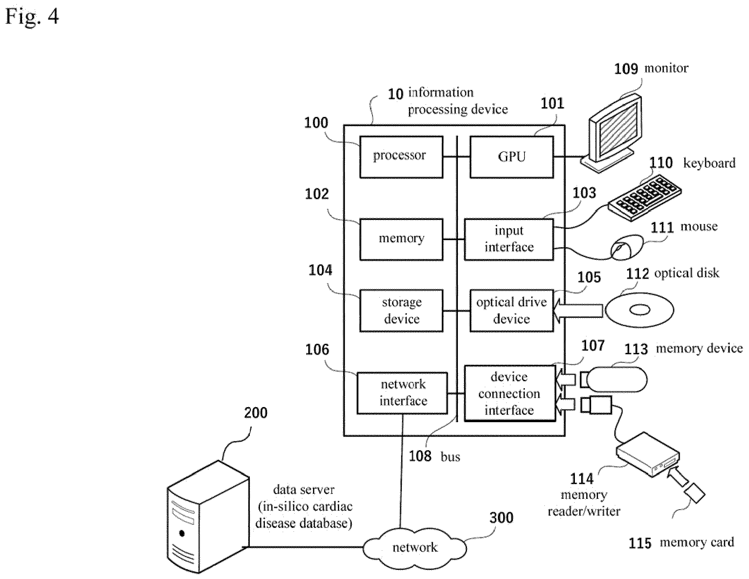

Fig. 4 is a drawing showing an example of the system configuration and the hardware configuration. In the example shown in Fig. 4, the information processing device 10 is connected to a data server 200 storing the in-silico cardiac disease database via a network 300. The entire information processing device 10 is controlled by a processor 100. The memory 102 and a plurality of peripheral devices are connected to the processor 100 via a bus 108. The processor 100 can be a multiprocessor. The processor 100 is, for example, a CPU (Central Processing Unit), MPU (Micro Processing Unit) or DSP (Digital Signal Processor). At least a part of the functions achieved by executing the programs by the processor 100 can be alternatively achieved by electronic circuits such as ASIC (Application Specific Integrated Circuit) and PLD (Programmable Logic Device).

The memory 102 is used as a main storage device of the storage unit 11. The memory 102 stores at least a part of the programs of OS (Operating System) and application programs executed by the processor 100. In addition, the memory 102 stores various data used for the processing executed by the processor 100. For example, a nonvolatile semiconductor memory device such as RAM (Random Access Memory) is used as the memory 102.

As for the examples of the peripheral devices connected to the bus 108, a storage device 104, a GPU (Graphics Processing Unit) 101, an input interface 103, an optical drive device 105, a device connection interface 107 and a network interface 106 can be listed.

The storage device 104 electrically or magnetically writes the data in a built-in recording medium and reads the data from the built-in recording medium. The storage device 104 is used as an auxiliary storage device of the information processing device 10. The storage device 104 stores the programs of OS, application programs and various data. Note that HDD (Hard Disk Drive) and SSD (Solid State Drive) can be used as the storage device 104, for example.

The GPU 101 is an arithmetic device for performing image processing and is also called a graphic controller. A monitor 109 is connected to the GPU 101. The GPU 101 makes the screen of the monitor 109 display the images in accordance with the instruction from the processor 100. As for the examples of the monitor 109, a display device using an organic EL (Electro Luminescence) and a liquid crystal display device can be listed.

A keyboard 110 and a mouse 111 are connected to the input interface 103. The input interface 103 transmits the signals transmitted from the keyboard 110 and the mouse 111 to the processor 100. Note that the mouse 111 is an example of a pointing device. The other pointing devices can be also used. As for the other pointing devices, a touch panel, a tablet, a touch pad and trackball can be listed.

The optical drive device 105 reads the data stored in an optical disk 112 or writes the data in the optical disk 112 using laser light or the like. The optical disk 112 is a portable recording medium for recording the data so as to be readable by the reflection of light. As for the optical disk 112, DVD (Digital Versatile Disc), DVD-RAM, CD-ROM (Compact Disc Read Only Memory), CD-R (Recordable)/RW (Re Writable) and the like can be listed.

The device connection interface 107 is a communication interface for connecting the peripheral devices to the information processing device 10. For example, a memory device 113 and a memory reader/writer 114 can be connected to the device connection interface 107. The memory device 113 is a recording medium having the function to communicate with the device connection interface 107. The memory reader/writer 114 is a device for writing the data in a memory card 115 or reading the data from the memory card 115. The memory card 115 is a card type recording medium.

The network interface 106 is connected to the network 300. The network interface 106 transmits and receives the data to/from the other computers or communication devices via the network 300. The network interface 106 is a wired communication interface connected to a wired communication device such as a switch and a router through a cable. In addition, the network interface 106 can be a wireless communication interface communicated and connected to a wireless communication device such as a base station or an access point through electric wave.

The information processing device 10 can achieve the processing function of the first embodiment by the above described hardware, for example.

When the processor 100 of the information processing device 10 has high performance and capacities of a memory 102 and a storage device 104 are enough, it is possible for the information processing device 10 to perform a large amount of cardiac simulation and create the in-silico cardiac disease database by the information processing device 10 itself.

[Second embodiment]

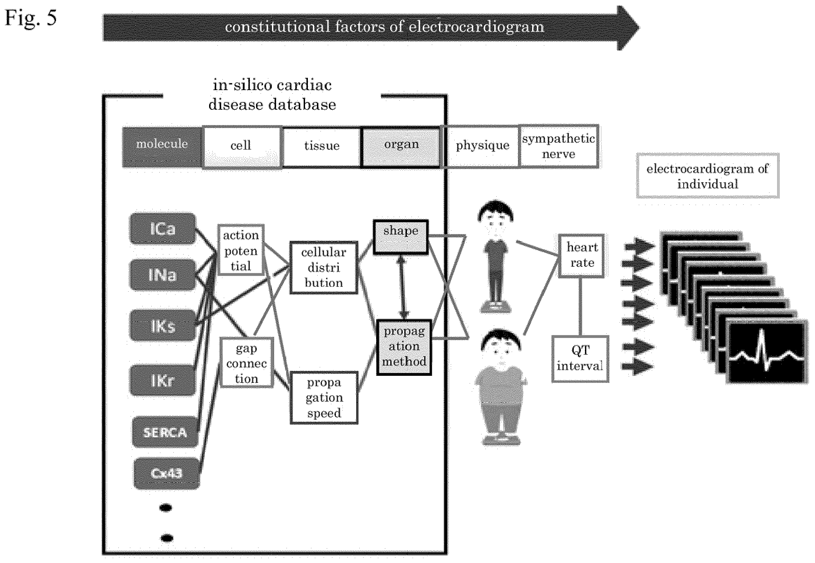

The second embodiment is a precise diagnosis related to the cardiac diseases of the individual based on a simple health check. The electrocardiogram and the echocardiography are frequently used in a regular health check and in an ordinary inspection. If the cardiac model having the electrocardiogram and the echocardiography in the in-silico cardiac disease database most approximate to the electrocardiogram and the echocardiography of the individual can be identified, a physiological variation of each factor related to the cardiac disease can be known for the individual. Thus, the information for the individual precise diagnosis and the individual medical treatment can be provided. Here, it should be noted that the in-silico cardiac disease database cannot be the database infinitely reflecting the diversity of the individual in principle. In particular, the following problem exists in the electrocardiogram. Fig. 5 shows an outline of constitutional factors of the electrocardiogram and a range covered by the in-silico cardiac disease database shown in Non-Patent Document 3 as an example of the range covered by the in-silico cardiac disease database. Here, the influence caused by the diversity of the physique and the sympathetic nerve are excluded from the variation factor of the database. Namely, in the in-silico cardiac disease database of the present example, a standard value is used for the above described factors. However, it is considered that the actual electrocardiogram of the individual is modified by the diversity of the above described factors. If the actual electrocardiogram of the individual is compared with the electrocardiogram in the in-silico cardiac disease database without the correction, there is a possibility that the electrocardiogram is diagnosed as illness even when the electrocardiogram is actually normal. The opposite situation may also occur. Therefore, it is required to correct the above described excluded factors. An example of a method of the correction is shown below.

First, the correction about the sympathetic nerve is performed as described below. The influence of the sympathetic nerve results in the variation of a heart rate. In the in-silico cardiac disease database, the electrocardiogram is based on the condition that the heart rate is 1 Hz (60 beats per minute). However, the actual heart rate varies depending on the influence of the sympathetic nerve. As a result, the QT interval, which is the time from Q wave to the end of T wave, is different although the QT interval is an important index. As for the method of correcting the QT interval, Bazett's formula shown below is widely used, for example. Based on the above described facts, the waveform of the actual electrocardiogram of the individual is digitalized and then adjusted in a time axis direction by computer programs.

Bazett's formula:

$$QT_C = \frac{QT}{\sqrt{RR}} \qquad (1)$$

In the above described formula, QT and RR mean the QT interval and an RR interval (one cardiac cycle) respectively. QTc is the QT interval after the correction.

When the time axis is corrected as described above, QRS width also varies at the same ratio. Thus, the QRS width is corrected by using the following formula in accordance with Non-Patent Document 4.

$$QRS_C = QRS + 0.0125 \times (1000 - RR) \qquad (2)$$

Here, QRSc and QRS mean the QRS width after the correction and the QRS width before the correction respectively.

Next, an example of the method for correcting the physique is shown. As the obesity advances, the diaphragm is lifted and the angle of the heart is changed toward the horizontal direction. Thus, the waveform of the electrocardiogram is affected by the change of the angle of the heart. Therefore, an anatomical axis of the heart is estimated from a chest X-ray image photographed in an ordinary health check. The cardiac model in the in-silico cardiac disease database is rotated to coincide with the anatomical axis and the electrocardiogram is recalculated by computer programs for all cases. The rotation of the cardiac model can be achieved only by converting the coordinates of the calculation lattice points constituting the cardiac model by the orthogonal tensor. In addition, the re-calculation of the electrocardiogram can be easily performed by using the following formula (3), for example, since the simulation result of electrical excitation propagation in the heart is stored in the in-silico cardiac disease database for all cases.

$$\Phi(r) = q/(4\pi\mu|r - r'|) \qquad (3)$$

The formula (3) is the formula for imparting a potential $\Phi(r)$ formed at a position vector r by an electrical charge q located at a position vector r' when a homogeneous infinite medium having a permittivity $\mu$ is assumed. Since momentarily varied source and sink current at each of the calculation lattice points r' in the cardiac muscle is calculated and stored by the cardiac simulation, the potential $\Phi(r)$ at the position vector r of the 12-lead electrocardiogram can be immediately calculated by evaluating the electrical charge q from the source and sink current.

In addition to the change of the angle of the heart, the influence of the increment of the distance between the electrodes and the heat caused by the increment of the thickness of the human body (thickness of the fat layer) is also considered. In this case, it is enough if the position vector r of the electrode in the formula (3) is corrected. In addition, it is also possible to correct the permittivity $\mu$ considering the fat layer if necessary.

In addition to the above described method, various methods can be considered for correcting the electrocardiogram about the physique. For example, instead of using the formula (3) assuming the homogeneous infinite medium, it is also possible to create a precise body model formed by further modeling skeleton and lung using the finite element method based on the chest X-ray image and the like in addition to the correction of the angle of the axis of the heart. In this case, the re-calculation of the electrocardiogram is the finite element analysis of the heterogeneous finite medium controlled by Poisson equation (formula (4)). The matrix obtained by discretizing the left-hand side of the formula is common in all cases and only the right-hand side f varies depending on the result of the cardiac simulation of each case. Thus, it is enough if the triangular factorization of the matrix is performed only at once and the calculation load is not excessive.

$$\nabla^2 \Phi(r) = f \qquad (4)$$

Here, $\nabla^2$ indicates Laplacian operator. Alternatively, on the contrary, it is also possible to correct the angle of the cardiac model more simply based on the statistic correlation between Body Mass Index (BMI) and the angle of the heart without using the chest X-ray image.

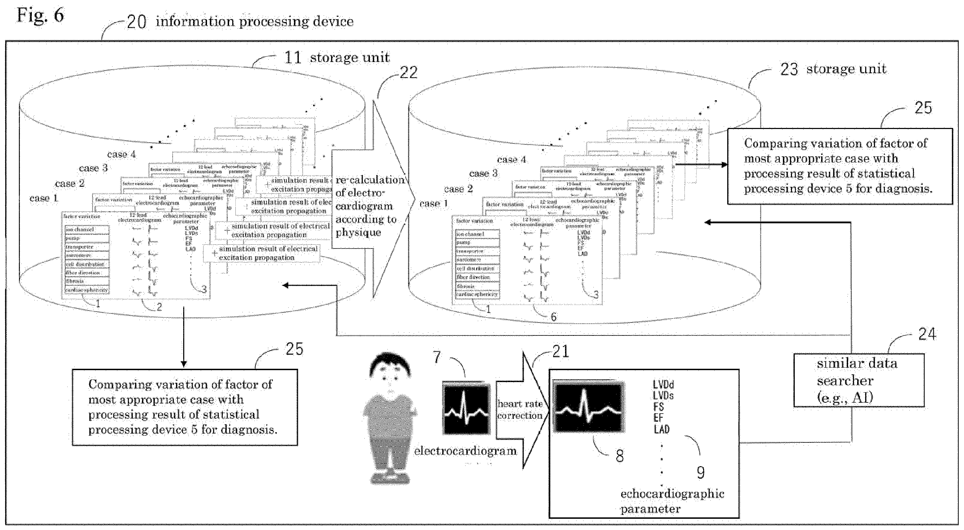

Fig. 6 shows an example of the second embodiment. Fig. 6 shows a functional configuration of the information processing device 20 which identifies the cardiac model having the electrocardiogram and the echocardiographic parameter in the in-silico cardiac disease database most approximate to the actual electrocardiogram and the actual echocardiographic parameter of a predetermined individual. The information processing device 20 is composed of a function 21 for correcting the heart rate of the actual electrocardiogram of the individual, a function 22 for correcting the in-silico cardiac disease database about the physique, a storage unit 23 for storing the data corrected by the function 22 in the storage unit 23 and a similar data searcher 24.

As described above, the correction 21 about the heart rate is performed on the actual electrocardiogram 7 of the predetermined individual to obtain the actual electrocardiogram 8 after the correction. On the other hand, the correction 22 about the physique is performed on the electrocardiogram 2 in the storage unit 11 and the electrocardiogram 2 is replaced with the amended electrocardiogram 6. A new data in which the electrocardiogram 2 is replaced with the electrocardiogram 6 is stored in the storage unit 23. The similar data searcher 24 identifies a case most approximate to a combination of the corrected actual electrocardiogram 8 and the corrected actual echocardiographic parameter 9 of the predetermined individual in the storage unit 23. AI can be used as the similar data searcher 24, for example. In this case, the AI can be a machine learning where the features are designated by a human or a deep learning. Furthermore, about the electrocardiogram, it is also possible to select the waveform having the mutual correlation coefficient nearer to 1 instead of using AI, for example. About the echocardiographic parameter, it is also possible to use a least-squares method where the square sum of the errors of each parameter (value) is minimized, for example. When the most approximate case is identified as described above, it is considered that the variation amount of each factor of the identified case indicates the physiological state of the predetermined individual. On the other hand, there is the variation distribution of the factors about various cardiac diseases processed in the statistical processing device of Fig. 2 (first embodiment). Thus, the diagnosis of the individual can be performed in association with the cardiac diseases by comparing the variation amount with the variation distribution.

In order to perform the correction 22 of the electrocardiogram 2 about the physique, the simulation result of the electrical excitation propagation of the hearts in the in-silico cardiac disease database is required. Therefore, in addition to the variation amount data 1, the electrocardiogram 2 and the echocardiographic parameter 3 of each factor, the simulation result of the electrical excitation propagation is also imported in the storage unit 11.

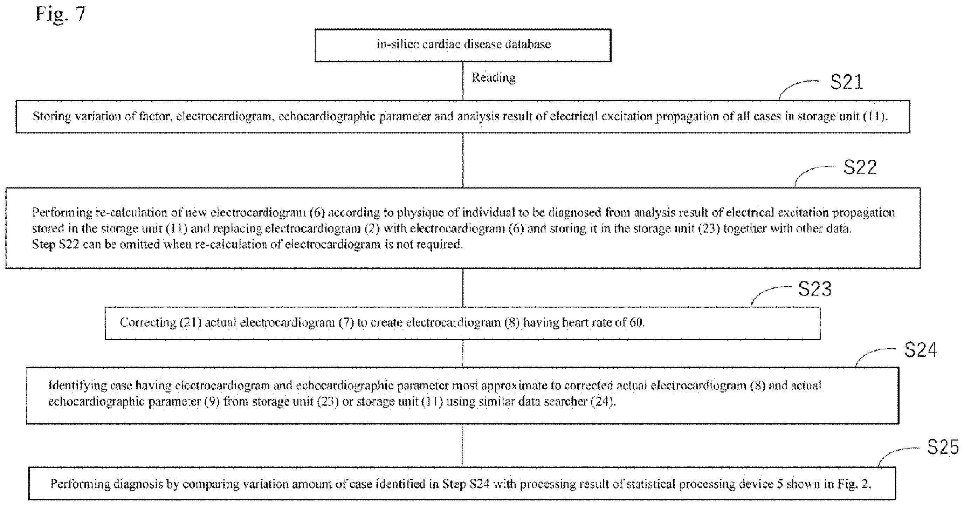

Fig. 7 is a flowchart showing an example of procedures of the above described information processing.

[Step S21] The variation amount of the factor, the electrocardiogram, the echocardiographic parameter and the analysis result of the electrical excitation propagation of all cases are read from the in-silico cardiac disease database and stored in the storage unit 11.

[Step S22] The re-calculation of new electrocardiogram 6 is performed according to the physique of the individual to be diagnosed in the processing 22 from the analysis result of the electrical excitation propagation stored in the storage unit 11 and the electrocardiogram 2 is replaced with the electrocardiogram 6 and stored in the storage unit 23 together with the other data. When the physique of the individual is close to a standard physique used for creating the in-silico cardiac disease database, the above described re-calculation of the electrocardiogram can be omitted.

[Step S23] The actual electrocardiogram 7 of the individual is corrected in the processing 21 and this is used as the actual electrocardiogram 8 having the heart rate of 60.

[Step S24] The case having the electrocardiogram and the echocardiographic parameter most approximate to the corrected actual electrocardiogram 8 and actual echocardiographic parameter 9 are identified using the similar data searcher 24. When the re-calculation of the electrocardiogram is performed, the data stored in the storage unit 23 searched. When the re-calculation of the electrocardiogram is not performed, the data stored in the storage unit 11 is searched.

[Step S25] The variation amount of each factor of the case identified in the Step S24 is compared with the processing result of the statistical processing device 5 shown in Fig. 2 (first embodiment). Thus, the diagnosis is performed.

Examples of the system configuration and the hardware configuration are same as Fig. 4 used in the first embodiment. When the processor 100 of the information processing device 10 has high performance and the capacities of the memory 102 and the storage device 104 are enough, it is possible for the information processing device 10 to perform a large amount of cardiac simulation and create the in-silico cardiac disease database by the information processing device 10 itself.

[Third embodiment]

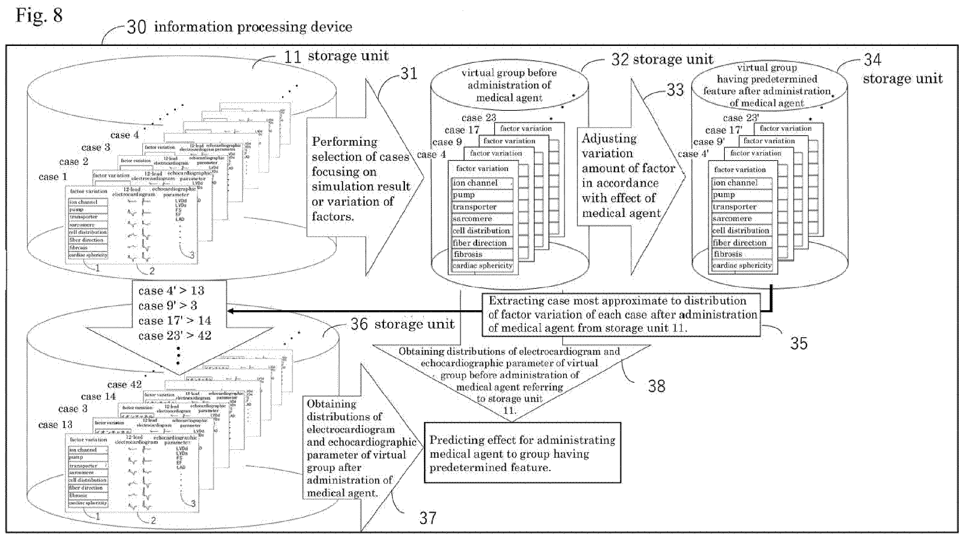

In the third embodiment, the group having a predetermined feature is intentionally extracted from the in-silico cardiac disease database as a subset focusing on the simulation result or the combination of the variation of the one or more factors and the effect of an administration of a medical agent to the group is easily evaluated. Fig. 8 is a drawing showing an example of the third embodiment. In the information processing device 30 shown in Fig. 8, a selection (and deletion) of the data is performed in the processing 31 from the data stored in the storage unit 11 into which an entire or a part of the data of the in-silico cardiac disease database is imported focusing on the simulation result or the combination of the variation of the one or more factors. The result is stored in a storage unit 32. It is enough if only the case number and the variation data of the factors are stored. As an example, when only the cases having long QT interval are extracted as the simulation result, the group of hearts having high risk of arrhythmia can be stored in the storage unit 32. As an example of focusing on each of the variation amount of each factor, the group of hearts having the trait distribution of a predetermined actual group (e.g., Japanese) can be stored in the storage unit 32 by extracting the simulation results so that the variation amount of each factor becomes a specified distribution. As an additional explanation of the latter example, since the ratio of the polymorphism distribution of Japanese related to the genes of KCNQ1, KCNE1, KCNH2 and SCN5A constituting ion channels responsible for the electrophysiologic property of the heart is known (Non-Patent Document 5), the frequency distribution of the variation amount of various ion channels can be identified based on the above described data as the variation factor. Then, the variation amount of each factor is adjusted in 33 assuming the case of administrating a predetermined medical agent to a virtual group stored in the storage unit 32 as described above. As a specific example, since how a predetermined medical agent inhibits or activates various ion channels in accordance with the density can be quantitatively measured by a patch clamp experiment (Non-Patent Document 6), the variation of the factors is adjusted in accordance with the above described data. For example, when the potassium current IKr before the administration of the medical agent is given divided into five stages of 100%, 90%, 80%, 70% and 60% as the variation of the factor of the KCNH2 channel for controlling the potassium current IKr and if the inhibition ratio of the channel by administrating the medical agent is 10% from the patch clamp experiment, the potassium current IKr after the administration of the medical agent is adjusted to five stages of 90%, 81%, 72%, 63% and 54%. As described above, a virtual group after the administration of the medical agent formed by adjusting the variation of each of the factors is stored in a storage unit 34. Finally, the case most approximate to the variation of the factor of each case stored in the storage unit 34 is extracted from the original group stored in the storage unit 11 in the processing 35, and the case is stored in a storage unit 36 together with accompanying electrocardiogram and echocardiographic parameter. On the other hand, a virtual group before the administration of the medical agent is stored in the storage unit 32, the distribution of the electrocardiogram and the echocardiographic parameter before the administration of the medical agent can be obtained by referring to the storage unit 11. Accordingly, the electrocardiogram and the echocardiographic parameter before and after the administration of the medical agent can be compared with each other in the processing 37, 38. Thus, the effect of administrating the medical agent to the group having a predetermined feature can be statistically evaluated. Note that the cardiac simulation requiring heavy calculation load is not required at all in the above described processing and the result can be easily obtained.

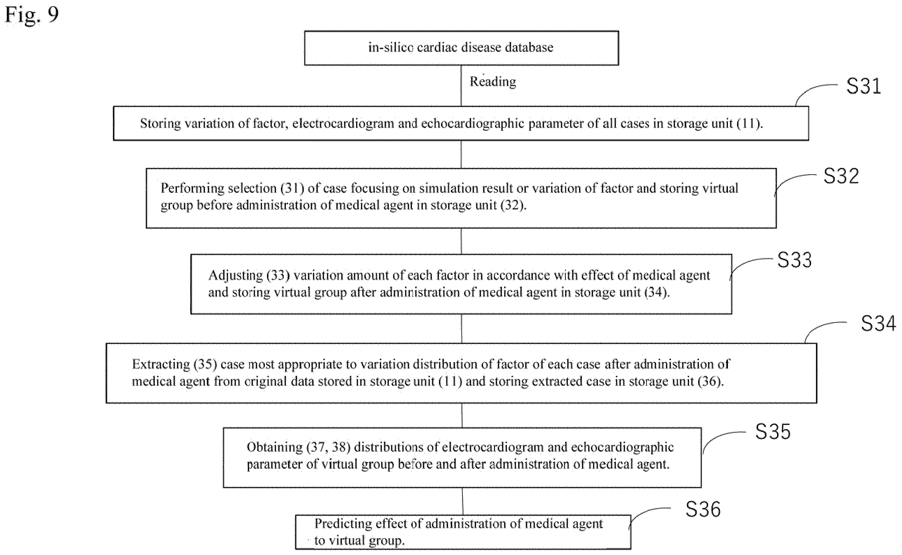

Fig. 9 is a flowchart showing an example of procedures of the above described information processing.

[Step S31] The variation of the factor, the electrocardiogram and the echocardiographic parameter of all cases are read from the in-silico cardiac disease database and stored in the storage unit 11.

[Step S32] The selection of cases in the storage unit 11 is performed focusing on the simulation result or the variation of the factor (processing 31) and the virtual group before the administration of the medical agent is stored in the storage unit 32.

[Step S33] The variation amount of each factor is adjusted in accordance with the effect of the medical agent in the processing 33 and the virtual group after the administration of the medical agent is stored in the storage unit 34.

[Step S34] The case most appropriate to the variation distribution of the factor of each case after the administration of the medical agent is extracted from the original data stored in the storage unit 11 in the processing 35 and the case is stored in a storage unit 36 together with accompanying electrocardiogram and echocardiographic parameter.

[Step S35] The distributions of the electrocardiogram and the echocardiographic parameter of the virtual group before and after the administration of the medical agent are obtained (processing 37, 38).

[Step S36] The distributions of the electrocardiogram and the echocardiographic parameter before and after the administration of the medical agent obtained in Step S35 are compared with other. Thus, the effect of the administration of the medical agent to a predetermined virtual group can be predicted.

Examples of the system configuration and the hardware configuration are same as Fig. 4 used in the first embodiment. When the processor 100 of the information processing device 10 has high performance and the capacities of the memory 102 and the storage device 104 are enough, it is possible for the information processing device 10 to perform a large amount of cardiac simulation and create the in-silico cardiac disease database by the information processing device 10 itself.

[Other embodiments]

In the first embodiment, the similar method can be applied for a medical equipment. An example is explained below.

An implantable cardioverter defibrillator (ICD) causes great psychological stress to the implanted patient since severe pain is caused during the defibrillation. However, many patients to which the ICD is implanted do not develop lethal arrhythmia and the ICD is not operated. Because of this, a method for determining adaptability of implanting the ICD has been researched. However, the research and the provocation test having sufficient sensitivity and specificity are not reported. Namely, unnecessary implantation of the ICD is currently performed. This causes unnecessary stress of the patient and the burden to the medical economy. On the other hand, the operation of the ICD is recorded for each patient to which the ICD is implanted. Accordingly, if the risk of operating the ICD can be judged only from the electrocardiogram and the echocardiographic parameter by the AI which learns the operation record of the ICD, the actual electrocardiogram and the actual echocardiographic parameter by the machine learning, it is possible to provide the information for identifying factors associated with the operation of the ICD, analyzing a mechanism and developing a reasonable therapeutic method in the similar method as the first embodiment. As another example of the medical equipment, a cardiac resynchronization therapy (CRT) can be listed. The implantation of the CRT is performed for improving the cardiac output by pacing both ventricles. However, it is reported that no effect could be seen in more than 30% of patients. Accordingly, if the effect of the implantation of the CRT can be judged only from the electrocardiogram and the echocardiographic parameter by the AI which learns a large number of records of patients to which the CRT is implanted showing the existence/absence of the effect, the actual electrocardiogram and the actual echocardiographic parameter by the machine learning, it is possible to provide the information for identifying factors associated with the effectiveness of the CRT, analyzing a mechanism and developing a reasonable therapeutic method in the similar method as the first embodiment.

Furthermore, the completely same method can be also applied to a therapeutic medicine without limited to the medical equipment. For example, if the existence/absence of the therapeutic effect of the therapeutic medicine can be judged only from the electrocardiogram and the echocardiographic parameter by the AI which learns the record of the existence/absence of the therapeutic effect of the therapeutic medicine in the actual clinical practice, the actual electrocardiogram and the actual echocardiographic parameter by the machine learning, it is possible to provide the information for identifying factors associated with the effectiveness of the therapeutic medicine, analyzing a mechanism and developing a reasonable therapeutic method in the similar method as the first embodiment.

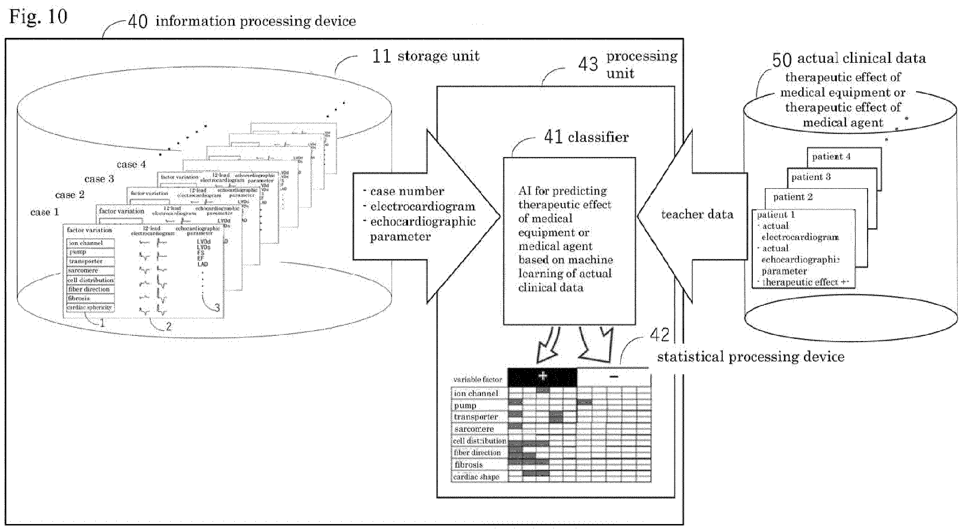

Since the above described examples are the common embodiment, these can be shown as the information processing device 40 of Fig. 10, for example. AI 41 capable of predicting the existence/absence of the therapeutic effect only from the electrocardiogram and the echocardiographic parameter is created by making the AI 41 learn a clinical data 50 composed of a large number of actual electrocardiograms and actual echocardiographic parameter and the record of the existence/absence of the therapeutic effect of the medical equipment or the medical agent as a teacher data. When the electrocardiogram 2 and the echocardiographic parameter 3 read from the in-silico cardiac disease database is inputted to the AI 41, the cases are classified into two groups of existence and absence of the therapeutic effect. By calculating the variation distribution of each factor in the above described two groups using a statistical processing device 42, it is possible to provide the information for identifying factors associated with the therapeutic effect, analyzing a mechanism and developing a reasonable therapeutic method.

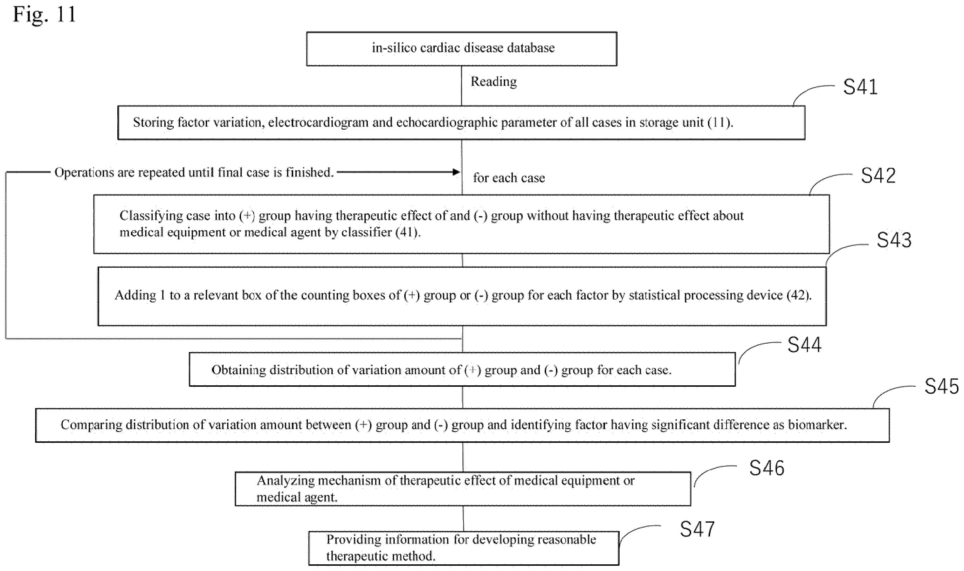

Fig. 11 is a flowchart showing an example of procedures of the above described information processing.

[Step S41] The variation 1 of the factor, the electrocardiogram 2 and the echocardiographic parameter 3 of all cases are read from the in-silico cardiac disease database and stored in the storage unit 11. The case number is assigned to each case.

[Step S42] The electrocardiogram 2 and the echocardiographic parameter 3 for each case is extracted from the storage unit 11 and inputted into a classifier 41 to classify the cases into the (+) group having the therapeutic effect of and the (-) group without having the therapeutic effect about the medical equipment or the medical agent.

[Step S43] In a statistical processing device 42, the counting boxes indicating the variation amount of each factor are prepared for each of the (+) group and the (-) group. For each factor, 1 is added to a relevant box of the counting boxes in any one of the (+) group and the (-) group.

Returning to Step S42, similar operations are repeated until the final case is finished.

[Step S44] A distribution of the values of the counting boxes of the (+) group and the values of the counting boxes of the (-) group, which is the distribution of the variation amount, can be obtained.

[Step S45] The distribution of the variation amount is compared between the (+) group and the (-) group and the factor having a significant difference is identified as a biomarker.

[Step S46] The mechanism of the therapeutic effect of the medical equipment or the medical agent is analyzed considering the causal relationship between the factors.

[Step S47] The information for developing the reasonable therapeutic method is provided from the biomarker identified in Step S45 and the therapeutic effect of the medical equipment or the medical agent analyzed in Step S46.

Examples of the system configuration and the hardware configuration are same as Fig. 4 used in the first embodiment. When the processor 100 of the information processing device 10 has high performance and the capacities of the memory 102 and the storage device 104 are enough, it is possible for the information processing device 10 to perform a large amount of cardiac simulation and create the in-silico cardiac disease database by the information processing device 10 itself.

[Description of the Reference Numerals]

1: variation data of various factors which may be associated with cardiac disease;
2: electrocardiogram obtained by simulation;
3: echocardiographic parameter obtained by simulation;
4: classifier based on automatic diagnosis;
5: statistical processing device for calculating trait distribution of variation amount of various factors;
6: electrocardiogram corrected according to physique;
7: actual electrocardiogram of individual;
8: actual electrocardiogram corrected according to heart rate;
9: actual echocardiographic parameter of individual;
10: information processing device for achieving first embodiment;
11: storage unit for storing data read from in-silico cardiac disease database;
12: processing unit for achieving first embodiment;
20: information processing device for achieving second embodiment
21: processing unit for correcting actual electrocardiogram according to heart rate;
22: processing unit for correcting electrocardiogram according to physique of individual;
23: storage unit for storing corrected data of electrocardiogram;
24: searcher for identifying data most appropriate to electrocardiogram or the like of individual from storage unit 11 or 23;
25: processing unit for comparing variation of factor of most appropriate case with processing result of statistical processing device 5 for diagnosis;
30: information processing device for achieving third embodiment;
31: processing unit for selecting case while focusing on simulation result or variation of factor;
32: storage unit for storing virtual group having predetermined feature before administration of medical agent;
33: processing unit for adjusting amount of variation of factor according to effect of medical agent;
34: storage unit for storing virtual group having predetermined feature after administration of medical agent;
35: processing unit for extracting case most approximate to variation distribution of factor of each case after adjustment from storage unit 11;
36: storage unit for storing case extracted by processing unit 35 and accompanying electrocardiogram and echocardiographic parameter;
37: processing unit for obtaining electrocardiogram of virtual group after administration of medical agent and distribution of echocardiographic parameter;
38: processing unit for obtaining distribution of electrocardiogram and echocardiographic parameter of virtual group before administration of medical agent;
40: information processing device for achieving other embodiments;
41: AI (classifier) for predicting therapeutic effect of medical equipment or medical agent based on machine learning of actual clinical data;
42: statistical processing device for calculating trait distribution of variation amount of various factors;
43: processing unit for achieving other embodiments;
50: clinical data composed of record of existence/absence of therapeutic effect of medical equipment or medical agent, actual electrocardiogram and actual echocardiographic parameter;
51: processing unit for machine learning of clinical data 50.

**Claims**

1. An information processing method (Fig. 2, Fig. 3) for making a computer execute steps of:

classifying a plurality of cases into two groups of positive and negative or into a plurality of positive groups depending on a degree of progress of a cardiac disease by inputting one or both of an electrocardiogram (2) and an echocardiographic parameter (3) obtained from an in-silico cardiac disease database into a classifier (4) which performs an automatic diagnosis of the cardiac disease;
performing a statistical processing (5) of a variation (1) of each factor which is associated with the electrocardiogram (2) and the echocardiographic parameter (3);
analyzing a feature of a distribution of a variation amount of each factor of the groups obtained from a result of the statistical processing (5);

identifying an influencing factor or influencing factors as a physiological biomarker associated with the cardiac disease;

analyzing a pathogenic mechanism of the cardiac disease from a causal relationship between the influencing factors; and

providing an information of developing a therapeutic method capable of suppressing a variation of the influencing factor or the influencing factors.

**2.** An information processing program (Fig. 2, Fig. 3) for making a computer execute steps of:

classifying a plurality of cases into two groups of positive and negative or into a plurality of positive groups depending on a degree of progress of a cardiac disease by inputting one or both of an electrocardiogram (2) and an echocardiographic parameter (3) obtained from an in-silico cardiac disease database into a classifier (4) which performs an automatic diagnosis of the cardiac disease;

performing a statistical processing (5) of a variation (1) of each factor which is associated with the electrocardiogram (2) and the echocardiographic parameter (3);

analyzing a feature of a distribution of a variation amount each factor of the groups obtained from a result of the statistical processing (5);

identifying an influencing factor or influencing factors as a physiological biomarker associated with the cardiac disease;

analyzing a pathogenic mechanism of the cardiac disease from a causal relationship between the influencing factors; and

providing an information of developing a therapeutic method capable of suppressing a variation of the influencing factor or the influencing factors.

**3.** An information processing device (10) (Fig. 2, Fig. 3, Fig. 4) including a storage unit and a processing unit, wherein

the processing unit is configured to classify a plurality of cases into two groups of positive and negative or into a plurality of positive groups depending on a degree of progress of a cardiac disease by inputting one or both of an electrocardiogram (2) and an echocardiographic parameter (3) obtained from an in-silico cardiac disease database into a classifier (4) which performs an automatic diagnosis of the cardiac disease,

the processing unit is configured to perform a statistical processing (5) of a variation (1) of each factor which is associated with the electrocardiogram (2) and the echocardiographic parameter (3),

the processing unit is configured to analyze a feature of a distribution of a variation amount of each factor of the groups obtained from a result of the statistical processing (5),

the processing unit is configured to identify an influencing factor or influencing factors as a physiological biomarker associated with the cardiac disease,

the processing unit is configured to analyze a pathogenic mechanism of the cardiac disease from a causal relationship between the influencing factors, and

the processing unit is configured to provide an information of developing a therapeutic method capable of suppressing a variation of the influencing factor or the influencing factors.

**4.** An information processing method (Fig. 6, Fig. 7) for making a computer execute steps of:

reading a variation (1) of factor, an electrocardiogram (2), an echocardiographic parameter (3) and an analysis result of an electrical excitation propagation of all cases from an in-silico cardiac disease database and storing the variation (1) of the factor, the electrocardiogram (2), the echocardiographic parameter (3) and the analysis result of the electrical excitation propagation in a storage unit (11);

replacing the electrocardiogram (2) with a new electrocardiogram (6) which is generated by a re-calculation (22) from the analysis result of the electrical excitation propagation according to a physique of the individual to be diagnosed if necessary and storing the electrocardiogram (6) with the other data in a storage unit (23) while performing a correction (21) of an actual electrocardiogram (7) of the individual as an electrocardiogram (8) having a heart rate of 60;

clarifying the variation of the factor of the individual by identifying a case having the electrocardiogram and the echocardiographic parameter most approximate to the electrocardiogram (8) and an actual echocardiographic parameter (9) in a storage unit (23) or the storage unit (11) using a similar data searcher (24); and

comparing the variation of the factor of the individual with a variation amount of each factor of the groups on which the statistical processing (5) is performed in claim 1.

**5.** An information processing program (Fig. 6, Fig. 7) for making a computer execute steps of:

reading a variation (1) of factor, an electrocardiogram (2), an echocardiographic parameter (3) and an analysis result of an electrical excitation propagation of all cases from an in-silico cardiac disease database and storing the variation (1) of the factor, the electrocardiogram (2), the echocardiographic parameter (3) and the analysis result of the electrical excitation propagation in a storage unit (11);
replacing the electrocardiogram (2) with a new electrocardiogram (6) which is generated by a re-calculation (22) from the analysis result of the electrical excitation propagation according to a physique of the individual to be diagnosed if necessary and storing the electrocardiogram (6) with the other data in a storage unit (23) while performing a correction (21) of an actual electrocardiogram (7) of the individual as an electrocardiogram (8) having a heart rate of 60;
clarifying the variation of the factor of the individual by identifying a case having the electrocardiogram and the echocardiographic parameter most approximate to the electrocardiogram (8) and an actual echocardiographic parameter (9) in a storage unit (23) or the storage unit (11) using a similar data searcher (24); and
comparing the variation of the factor of the individual with a variation amount of each factor of the groups on which the statistical processing (5) is performed in claim 1.

**6.** An information processing device (20) (Fig. 4, Fig. 6, Fig. 7) including a storage unit and a processing unit and an information processing device, wherein

the processing unit is configured to read a variation (1) of factor, an electrocardiogram (2), an echocardiographic parameter (3) and an analysis result of an electrical excitation propagation of all cases from an in-silico cardiac disease database and store the variation (1) of the factor, the electrocardiogram (2), the echocardiographic parameter (3) and the analysis result of the electrical excitation propagation in a storage unit (11),
the processing unit is configured to replace the electrocardiogram (2) with a new electrocardiogram (6) which is generated by a re-calculation (22) from the analysis result of the electrical excitation propagation according to a physique of the individual to be diagnosed if necessary and store the electrocardiogram (6) with the other data in a storage unit (23) while performing a correction (21) of an actual electrocardiogram (7) of the individual as an electrocardiogram (8) having a heart rate of 60,
the processing unit is configured to clarify the variation of the factor of the individual by identifying a case having the electrocardiogram and the echocardiographic parameter most approximate to the electrocardiogram (8) and an actual echocardiographic parameter (9) in a storage unit (23) or the storage unit (11) using a similar data searcher (24), and
the processing unit is configured to compare the variation of the factor of the individual with a variation amount of each factor of the groups on which the statistical processing (5) is performed in claim 1.

**7.** An information processing method (Fig. 8, Fig. 9) for making a computer execute steps of:

reading a variation (1) of factor, an electrocardiogram (2) and an echocardiographic parameter (3) of all cases from an in-silico cardiac disease database and storing the variation (1) of the factor, the electrocardiogram (2) and the echocardiographic parameter (3) in a storage unit (11);
performing a selection (31) of the all cases stored in the storage unit (11) while focusing on a simulation result or the variation of the factor and storing a virtual group before an administration of a medical agent in a storage unit (32);
storing a virtual group after the administration of the medical agent in a storage unit (34) while performing an adjustment (33) of the variation of the factor stored in the storage unit (32) by an effect of the medical agent;
performing an extraction (35) of a case most approximate to the distribution of the variation of the factor of the case to which the adjustment (33) is performed from original data stored in the storage unit (11) and storing the case in a storage unit (36); and
performing an obtainment (37, 38) and comparing the distribution of the electrocardiogram and the echocardiographic parameter of the virtual group before and after the administration of the medical agent.

**8.** An information processing program (Fig. 8, Fig. 9) for making a computer execute steps of:

reading a variation (1) of factor, an electrocardiogram (2) and an echocardiographic parameter (3) of all cases from an in-silico cardiac disease database and storing the variation (1) of the factor, the electrocardiogram (2) and the echocardiographic parameter (3) in a storage unit (11);
performing a selection (31) of the all cases stored in the storage unit (11) while focusing on a simulation result or the variation of the factor and storing a virtual group before an administration of a medical agent in a storage unit (32);

storing a virtual group after the administration of the medical agent in a storage unit (34) while performing an adjustment (33) of the variation of the factor stored in the storage unit (32) by an effect of the medical agent; performing an extraction (35) of a case most approximate to the distribution of the variation of the factor of the case to which the adjustment (33) is performed from original data stored in the storage unit (11) and storing the case in a storage unit (36); and

performing an obtainment (37, 38) and comparing the distribution of the electrocardiogram and the echocardiographic parameter of the virtual group before and after the administration of the medical agent.

9. An information processing device (30) (Fig. 4, Fig. 8, Fig. 9) including a storage unit and a processing unit and an information processing device, wherein

the processing unit is configured to read a variation (1) of factor, an electrocardiogram (2) and an echocardiographic parameter (3) of all cases from an in-silico cardiac disease database and store the variation (1) of the factor, the electrocardiogram (2) and the echocardiographic parameter (3) in a storage unit (11),

the processing unit is configured to perform a selection (31) of the all cases stored in the storage unit (11) while focusing on a simulation result or the variation of the factor and storing a virtual group before an administration of a medical agent in a storage unit (32),

the processing unit is configured to store a virtual group after the administration of the medical agent in a storage unit (34) while performing an adjustment (33) of a variation amount of each factor stored in the storage unit (32) by an effect of the medical agent,

the processing unit is configured to perform an extraction (35) of a case most approximate to the distribution of the variation (1) of the factor of the case to which the adjustment (33) is performed from original data stored in the storage unit (11) and store the case in a storage unit (36), and

the processing unit is configured to perform an obtainment (37, 38) and compare the distribution of the electrocardiogram and the echocardiographic parameter of the virtual group before and after the administration of the medical agent.

10. An information processing method (Fig. 10, Fig. 11) for making a computer execute steps of:

classifying a plurality of cases into a first group where a therapeutic effect of a medical equipment or a medical agent is expected and a second group where the therapeutic effect is not expected or into a plurality of groups depending on a degree of an expected therapeutic effect based on a machine learning of an actual clinical data by inputting one or both of an electrocardiogram (2) and an echocardiographic parameter (3) obtained from an in-silico cardiac disease database into an AI (41) which can predict an existence of the therapeutic effect of the medical equipment or the medical agent;

performing a statistical processing (42) of a variation (1) of factor associated with the electrocardiogram (2) and the echocardiographic parameter (3);

identifying an influencing factor or influencing factors as a physiological biomarker associated with the therapeutic effect of the medical equipment or the medical agent by analyzing a feature of a distribution of the variation of the factor obtained from a result of the statistical processing;

analyzing a mechanism of the therapeutic effect of the medical equipment or the medical agent from a causal relationship between the influencing factors; and

providing an information of developing a therapeutic method capable of suppressing a variation of the influencing factor or the influencing factors.

11. An information processing program (Fig. 10, Fig. 11) for making a computer execute steps of:

classifying a plurality of cases into a first group where a therapeutic effect of a medical equipment or a medical agent is expected and a second group where the therapeutic effect is not expected or into a plurality of groups depending on a degree of an expected therapeutic effect based on a machine learning of an actual clinical data by inputting one or both of an electrocardiogram (2) and an echocardiographic parameter (3) obtained from an in-silico cardiac disease database into an AI (41) which can predict an existence of the therapeutic effect of the medical equipment or the medical agent;

performing a statistical processing (42) of a variation (1) of factor associated with the electrocardiogram (2) and the echocardiographic parameter (3);

identifying an influencing factor or influencing factors as a physiological biomarker associated with the therapeutic effect of the medical equipment or the medical agent by analyzing a feature of a distribution of the variation of the factor obtained from a result of the statistical processing;

analyzing a mechanism of the therapeutic effect of the medical equipment or the medical agent from a causal relationship between the influencing factors; and

providing an information of developing a therapeutic method capable of suppressing a variation of the influencing factor or the influencing factors.

12. An information processing device (40) (Fig. 4, Fig. 10, Fig. 11) including a storage unit and a processing unit and an information processing device, wherein

the processing unit is configured to classify a plurality of cases into a first group where a therapeutic effect of a medical equipment or a medical agent is expected and a second group where the therapeutic effect is not expected or into a plurality of groups depending on a degree of an expected therapeutic effect based on a machine learning of an actual clinical data by inputting one or both of an electrocardiogram (2) and an echocardiographic parameter (3) obtained from an in-silico cardiac disease database into an AI (41) which can predict an existence of the therapeutic effect of the medical equipment or the medical agent,

the processing unit is configured to performing a statistical processing (42) of a variation (1) of factor associated with the electrocardiogram (2) and the echocardiographic parameter (3),

the processing unit is configured to identify an influencing factor or influencing factors as a physiological biomarker associated with the therapeutic effect of the medical equipment or the medical agent by analyzing a feature of a distribution of the variation of the factor obtained from a result of the statistical processing,

the processing unit is configured to analyze a mechanism of the therapeutic effect of the medical equipment or the medical agent from a causal relationship between the influencing factors, and

the processing unit is configured to provide an information of developing a therapeutic method capable of suppressing a variation of the influencing factor or the influencing factors.

## Fig. 1

A cardiac simulation is performed for a large number of combinations by a massively parallel computation achieved by super computers where various macro and micro variable factors associated with cardiac diseases are variously changed.

| |
|---|
| ion channel |
| pump |
| transporter |
| sarcomere |
| cell distribution |
| fiber direction |
| fibrosis |
| cardiac shape |

examples of
variable factor

pulmonary
circulation circuit

R2p    C1p   R1p

R4p

LA

C2p   R3p

RA

R4a

R3a   C2a   R2a

cell distribution
fiber direction
fibrosis
cardiac shape

heart simulator

pulmonary
circulation circuit

R1a

sarcomere

Na⁺ Ca²⁺ K⁺

Ca²⁺   SR

C1a

ion channel
pump
transporter

## in-silico cardiac disease database

Simulation results associated with information of variation amount of each factor are stored.

electrocar
diogram

pressure-volume
relationship

EP 4 350 711 A1

Fig. 2

Fig. 3

| in-silico cardiac disease database |
|---|

Reading ⟋ S11

| Storing factor variation, electrocardiogram and echocardiographic parameter of all cases in storage unit (11). |
|---|

Operations are repeated until final case is finished. ⟶ for each case ⟋ S12

| Classifying cases into groups of existence (+) and absence (-) of causing heart failure by classifier (4). |
|---|

⟋ S13

| Adding 1 to relevant box of counting boxes of (+) group and (-) group for each factor by statistical processing device (5). |
|---|

⟋ S14

| Obtaining distribution of variation amount of (+) group and (-) group for each factor. |
|---|

⟋ S15

| Comparing distribution of variation amount between (+) group and (-) group and identifying factor having significant difference as biomarker. |
|---|

⟋ S16

| Analyzing mechanism of heart failure. |
|---|

⟋ S17

| Providing information for developing reasonable therapeutic method. |
|---|

EP 4 350 711 A1

19

Fig. 4

Fig. 5

EP 4 350 711 A1

**Fig. 6** 20 information processing device

11 storage unit

23 storage unit

25

case 4
case 3
case 2
case 1

re-calculation of electrocardiogram according to physique

Comparing variation of factor of most appropriate case with processing result of statistical processing device 5 for diagnosis.

25

Comparing variation of factor of most appropriate case with processing result of statistical processing device 5 for diagnosis.

similar data searcher (e.g., AI)

24

electrocardiogram

heart rate correction

LVDd
LVDs
FS
EF
LAD

echocardiographic parameter

## Fig. 7

```
                    ┌──────────────────────────────────────┐
                    │      in-silico cardiac disease database │
                    └──────────────────────────────────────┘
                                      │ Reading
```

**S21**

Storing variation of factor, electrocardiogram, echocardiographic parameter and analysis result of electrical excitation propagation of all cases in storage unit (11).

**S22**

Performing re-calculation of new electrocardiogram (6) according to physique of individual to be diagnosed from analysis result of electrical excitation propagation stored in the storage unit (11) and replacing electrocardiogram (2) with electrocardiogram (6) and storing it in the storage unit (23) together with other data.
Step S22 can be omitted when re-calculation of electrocardiogram is not required.

**S23**

Correcting (21) actual electrocardiogram (7) to create electrocardiogram (8) having heart rate of 60.

**S24**

Identifying case having electrocardiogram and echocardiographic parameter most approximate to corrected actual electrocardiogram (8) and actual echocardiographic parameter (9) from storage unit (23) or storage unit (11) using similar data searcher (24).

**S25**

Performing diagnosis by comparing variation amount of case identified in Step S24 with processing result of statistical processing device 5 shown in Fig. 2.

Fig. 8

30 information processing device

Performing selection of cases focusing on simulation result or variation of factors.

11 storage unit

case 1 / case 2 / case 3 / case 4

factor variation / 12-lead electrocardiogram / echocardiographic parameter

ion channel / pump / transporter / sarcomere / cell distribution / fiber direction / fibrosis / cardiac sphericity

31

Adjusting variation amount of factor in accordance with effect of medical agent

32 storage unit

virtual group before administration of medical agent

case 4 / case 9 / case 17 / case 23

factor variation

ion channel / pump / transporter / sarcomere / cell distribution / fiber direction / fibrosis / cardiac sphericity

33

34 storage unit

virtual group having predetermined feature after administration of medical agent

case 4' / case 9' / case 17' / case 23'

factor variation

ion channel / pump / transporter / sarcomere / cell distribution / fiber direction / fibrosis / cardiac sphericity

35

Extracting case most approximate to distribution of factor variation of each case after administration of medical agent from storage unit 11.

36 storage unit

case 4' > 13
case 9' > 3
case 17' > 14
case 23' > 42

case 13 / case 3 / case 14 / case 42

factor variation / 12-lead electrocardiogram / echocardiographic parameter

ion channel / pump / transporter / sarcomere / cell distribution / fiber direction / fibrosis / cardiac sphericity

Obtaining distributions of electrocardiogram and echocardiographic parameter of virtual group after administration of medical agent.

37

Obtaining distributions of electrocardiogram and echocardiographic parameter of virtual group before administration of medical agent referring to storage unit 11.

38

Predicting effect for administrating medical agent to group having predetermined feature.

Fig. 9

EP 4 350 711 A1

```
┌─────────────────────────────────────────────────────────────┐
│               in-silico cardiac disease database             │
└─────────────────────────────────────────────────────────────┘
                         │
                      Reading                              S31
┌─────────────────────────────────────────────────────────────────────────────┐
│ Storing variation of factor, electrocardiogram and echocardiographic parameter of all cases in storage unit (11). │
└─────────────────────────────────────────────────────────────────────────────┘
                         │
                                                          S32
┌─────────────────────────────────────────────────────────────────────────┐
│ Performing selection (31) of case focusing on simulation result or variation of factor and storing virtual │
│ group before administration of medical agent in storage unit (32).        │
└─────────────────────────────────────────────────────────────────────────┘
                         │
                                                          S33
┌───────────────────────────────────────────────────────────────────┐
│ Adjusting (33) variation amount of each factor in accordance with effect of medical agent │
│ and storing virtual group after administration of medical agent in storage unit (34). │
└───────────────────────────────────────────────────────────────────┘
                         │
                                                          S34
┌───────────────────────────────────────────────────────────────────────────────┐
│ Extracting (35) case most appropriate to variation distribution of factor of each case after administration of │
│ medical agent from original data stored in storage unit (11) and storing extracted case in storage unit (36). │
└───────────────────────────────────────────────────────────────────────────────┘
                         │
                                                          S35
┌─────────────────────────────────────────────────────────────────┐
│ Obtaining (37, 38) distributions of electrocardiogram and echocardiographic │
│ parameter of virtual group before and after administration of medical agent. │
└─────────────────────────────────────────────────────────────────┘
                         │
                                                          S36
┌─────────────────────────────────────────────┐
│ Predicting effect of administration of medical agent │
│ to virtual group.                            │
└─────────────────────────────────────────────┘
```

Fig. 10

40 information processing device

11 storage unit

43 processing unit

50 actual clinical data
therapeutic effect of medical equipment or therapeutic effect of medical agent

case 4
case 3
case 2
case 1

factor variation
12-lead electrocardiogram
echocardiographic parameter

factor variation
ion channel
pump
transporter
sarcomere
cell distribution
fiber direction
fibrosis
cardiac sphericity

LVDd
LVDs
FS
EF
LAD

1    2    3

· case number
· electrocardiogram
· echocardiographic parameter

41 classifier

AI for predicting therapeutic effect of medical equipment or medical agent based on machine learning of actual clinical data

teacher data

patient 4
patient 3
patient 2
patient 1
· actual electrocardiogram
· actual echocardiographic parameter
· therapeutic effect +

42

statistical processing device

| variable factor | + | | | − | | |
|---|---|---|---|---|---|---|
| ion channel | | | | | | |
| pump | | | | | | |
| transporter | | | | | | |
| sarcomere | | | | | | |
| cell distribution | | | | | | |
| fiber direction | | | | | | |
| fibrosis | | | | | | |
| cardiac shape | | | | | | |

EP 4 350 711 A1

Fig. 11

```
                    ┌────────────────────────────────────────────┐
                    │        in-silico cardiac disease database    │
                    └────────────────────────────────────────────┘
                                     │ Reading
                                                                        ─── S41
┌──────────────────────────────────────────────────────────────────────────────┐
│ Storing factor variation, electrocardiogram and echocardiographic parameter of │
│ all cases in storage unit (11).                                                │
└──────────────────────────────────────────────────────────────────────────────┘

── Operations are repeated until final case is finished. ──►  for each case
                                                                        ─── S42
┌──────────────────────────────────────────────────────────────────────────────┐
│ Classifying case into (+) group having therapeutic effect of and (-) group     │
│ without having therapeutic effect about medical equipment or medical agent by   │
│ classifier (41).                                                               │
└──────────────────────────────────────────────────────────────────────────────┘
                                                                        ─── S43
┌──────────────────────────────────────────────────────────────────────────────┐
│ Adding 1 to a relevant box of the counting boxes of (+) group or (-) group for  │
│ each factor by statistical processing device (42).                             │
└──────────────────────────────────────────────────────────────────────────────┘
                                                                        ─── S44
┌──────────────────────────────────────────────────────────────────────────────┐
│ Obtaining distribution of variation amount of (+) group and (-) group for each  │
│ case.                                                                          │
└──────────────────────────────────────────────────────────────────────────────┘
                                                                        ─── S45
┌──────────────────────────────────────────────────────────────────────────────┐
│ Comparing distribution of variation amount between (+) group and (-) group and  │
│ identifying factor having significant difference as biomarker.                 │
└──────────────────────────────────────────────────────────────────────────────┘
                                                                        ─── S46
┌──────────────────────────────────────────────────────────────────────────────┐
│ Analyzing mechanism of therapeutic effect of medical equipment or medical       │
│ agent.                                                                         │
└──────────────────────────────────────────────────────────────────────────────┘
                                                                        ─── S47
┌──────────────────────────────────────────────────────────────────────────────┐
│ Providing information for developing reasonable therapeutic method.             │
└──────────────────────────────────────────────────────────────────────────────┘
```

## INTERNATIONAL SEARCH REPORT

| International application No. |
| --- |
| **PCT/JP2022/032302** |

**A. CLASSIFICATION OF SUBJECT MATTER**

*G16H 50/50*(2018.01)i
FI: G16H50/50

According to International Patent Classification (IPC) or to both national classification and IPC

**B. FIELDS SEARCHED**

Minimum documentation searched (classification system followed by classification symbols)

G16H50/50

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2022/106580 A1 (ELEM BIOTECH S.L.) 27 May 2022 (2022-05-27) paragraphs [0149]-[0151], [0162] | 1-12 |
| A | JP 2021-522052 A (VEKTOR MEDICAL, INC.) 30 August 2021 (2021-08-30) paragraph [0017] | 1-12 |
| A | JP 2007-507814 A (ENTELOS, INC) 29 March 2007 (2007-03-29) paragraphs [0058], [0086]-[0093], [0127] | 1-12 |
| A | JP 2009-515602 A (IMMERSION CORPORATION) 16 April 2009 (2009-04-16) entire text, all drawings | 1-12 |
| A | JP 2022-517555 A (THE REGENTS OF THE UNIVERSITY OF CALIFORNIA) 09 March 2022 (2022-03-09) paragraphs [0057]-[0067] | 1-12 |
| A | JP 2022-512080 A (CARIS MPI, INC.) 02 February 2022 (2022-02-02) entire text, all drawings | 1-12 |

☐ Further documents are listed in the continuation of Box C.　　✓ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **27 October 2022** | **08 November 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

EP 4 350 711 A1

**INTERNATIONAL SEARCH REPORT**

| International application No. |
|---|
| **PCT/JP2022/032302** |

**Box No. III**     **Observations where unity of invention is lacking (Continuation of item 3 of first sheet)**

This International Searching Authority found multiple inventions in this international application, as follows:

Document 1: WO 2022/106580 A1 (ELEM BIOTECH S.L.) 27 May 2022 (2022-05-27) paragraphs [0149]-[0151], [0162] (Family: none)

Claims are classified into the following three inventions.

(Invention 1) Claims 1-6

Claims 1-6 have the special technical feature of "classifying into two groups of positive and negative, by inputting cases into a classifier (4) that automatically diagnoses heart disease, or classifying the cases into multiple positive groups depending on the degree of progression of heart disease and then statistically processing (5) the factor variation (1) related to the electrocardiogram (2) and the echocardiogram index (3); identifying influencing factors or influencing factor groups involved in heart disease as a physiological biomarker by analyzing the characteristics of the variation distribution of each factor in each group obtained from the statistical processing results; analyzing the onset mechanism of heart disease from the causal relationship between the influencing factors; and providing information for developing treatments that suppress variations in the influencing factors or influencing factor groups," and are thus classified as invention 1.

(Invention 2) Claims 7-9

Claims 7-9 share, with claim 1 classified as invention 1, the common technical feature of "reading the electrocardiogram (2) and echocardiogram index (3) from the in silico heart disease database." However, said technical feature does not make a contribution over the prior art in light of the disclosure of document 1 (in particular, see paragraphs [0149]-[0151], [0162]) and thus cannot be considered a special technical feature. Also, there are no other same or corresponding special technical features between claims 7-9 and claim 1.

In addition, claims 7-9 are not dependent on claim 1. Furthermore, claims 7-9 are not substantially identical to or similarly closely related to any of the claims classified as invention 1.

Therefore, claims 7-9 cannot be classified as invention 1.

Also, claims 7-9 have the special technical feature of "reading the factor variation (1) of all cases and storing the same in a storage unit (11); selecting (31) each case in the storage unit (11) by focusing on simulation results or factor variations; storing a virtual population before dosing in a storage unit (32); storing, in the storage unit (34), a virtual population after dosing, in which the amount of variation of each factor stored in the storage unit (32) is adjusted (33) according to the effect of the drug; extracting (35) a case, which most approximates the factor variation distribution of each case after adjustment from the raw data stored in the storage unit (11), and storing the case in the storage unit (36); and obtaining (37, 38) and comparing the distribution of electrocardiograms and echocardiographic indicators in a virtual population before and after dosing," and are thus classified as invention 2.

(Invention 3) Claims 10-12

Claims 10-12 share, with claim 1 classified as invention 1 and claim 7 classified as invention 2, the common technical feature of "electrocardiogram (2) and/or echocardiogram index (3) obtained from the in silico heart disease database." However, said technical feature does not make a contribution over the prior art in light of the disclosure of document 1 (in particular, see paragraphs [0149]-[0151], [0162]) and thus cannot be considered a special technical feature. In addition, there are no other same or corresponding special technical features between claims 10-12 and claim 1 or 7.

Also, claims 10-12 are not dependent on any of claim 1 or 7. Furthermore, claims 10-12 are not substantially identical to or similarly closely related to any of the claims classified as invention 1 or 2.

Therefore, claim 10-12 cannot be classified as either invention 1 or 2.

Also, claims 10-12 have the special technical feature of "inputting into an AI (41) that can predict whether a certain medical device or drug has a therapeutic effect on the basis of machine learning of actual clinical data; classifying groups into two groups, such as a group in which the therapeutic effect of the medical device or drug is expected and a group in which the therapeutic effect is not expected or classifying the groups into a plurality of groups according to the degree of predicted treatment effect and then statistically processing (42) the factor variation (1) related to the electrocardiogram (2) and the echocardiogram index (3); identifying influencing factors or influencing factor groups involved in the therapeutic effect of the medical device or drug as a physiological biomarker by analyzing the characteristics of the variation distribution of factors in each group obtained from the statistical processing results; analyzing the mechanism of the therapeutic effect of the medical device or drug from the causal relationship between the influencing factors; and providing information for developing treatments that suppress variations in the influencing factors or influencing factor groups," and are thus classified as invention 3.

Form PCT/ISA/210 (continuation of first sheet) (January 2015)

29

**INTERNATIONAL SEARCH REPORT**

International application No.

**PCT/JP2022/032302**

| | |
|---|---|
| **Box No. III** | **Observations where unity of invention is lacking (Continuation of item 3 of first sheet)** |

1. [✓] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**    [ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[✓] No protest accompanied the payment of additional search fees.

Form PCT/ISA/210 (continuation of first sheet) (January 2015)

# EP 4 350 711 A1

**INTERNATIONAL SEARCH REPORT**
**Information on patent family members**

International application No.

**PCT/JP2022/032302**

| Patent document cited in search report | | | Publication date (day/month/year) | Patent family member(s) | | | Publication date (day/month/year) |
|---|---|---|---|---|---|---|---|
| WO | 2022/106580 | A1 | 27 May 2022 | (Family: none) | | | |
| JP | 2021-522052 | A | 30 August 2021 | US | 2019/0333641 | A1 | |
| | | | | paragraph [0058] | | | |
| | | | | WO | 2019/210092 | A1 | |
| | | | | CN | 112437964 | A | |
| | | | | KR | 10-2021-0016359 | A | |
| JP | 2007-507814 | A | 29 March 2007 | US | 2005/0131663 | A1 | |
| | | | | paragraphs [0068], [0098]-[0105], [0143] | | | |
| | | | | WO | 2005/036446 | A2 | |
| | | | | CA | 2540280 | A | |
| JP | 2009-515602 | A | 16 April 2009 | US | 2007/0124128 | A1 | |
| | | | | whole document | | | |
| | | | | WO | 2007/059172 | A2 | |
| | | | | CN | 101310249 | A | |
| | | | | CN | 102662597 | A | |
| | | | | JP | 2012-230394 | A | |
| JP | 2022-517555 | A | 09 March 2022 | US | 2022/0061732 | A1 | |
| | | | | paragraphs [0078]-[0088] | | | |
| | | | | WO | 2020/142539 | A1 | |
| | | | | KR | 10-2021-0146883 | A | |
| | | | | CN | 113939243 | A | |
| JP | 2022-512080 | A | 02 February 2022 | US | 2021/0295979 | A1 | |
| | | | | whole document | | | |
| | | | | US | 2022/0262494 | A1 | |
| | | | | WO | 2020/113237 | A1 | |
| | | | | WO | 2021/112918 | A1 | |
| | | | | KR | 10-2021-0111254 | A | |

Form PCT/ISA/210 (patent family annex) (January 2015)

31

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6300244 B
- JP 2017033227 A
- JP 2022040892 A

**Non-patent literature cited in the description**

- **KARIYA T ; WASHIO T ; OKADA J ; NAKAGAWA M ; WATANABE M ; KADOOKA Y ; SANO S ; NAGAI R ; SUGIURA S ; HISADA T.** Personalized Perioperative Multi-scale, Multi-physics Heart Simulation of Double Outlet Right Ventricle. *Annals of Biomedical Engineering,* 2020
- **OKADA J ; WASHIO T ; NAKAGAWA M ; WATANABE M ; KADOOKA Y ; KARIYA T ; YAMASHITA H ; YAMADA Y ; MOMOMURA S ; NAGAI R.** Multi-scale, tailor-made heart simulation can predict the effect of cardiac resynchronization therapy. *Journal of Molecular and Cellular Cardiology.,* 2017, vol. 108, 17-23
- Research Organization for Information Science and Technology, high-performance general purpose computer high level utilization project in 2020, Fugaku Achievement Acceleration Program. *Overcoming heart failure pandemic with innovative integration of multi-scale heart simulator and large-scale clinical data*
- **MASON J. W ; STRAUS D. G ; VAGLIO M ; BADILINI F.** Correction of the QRS duration for the heart rate. *I Electrocardiol,* 2019, vol. 1-4
- **H. IWASA ; T. ITOH ; R. NAGAI ; Y. NAKAMURA ; T. TANAKA.** Twenty single nucleotide polymorphisms (SNPs) and their allelic frequencies in four genes that are responsible for familial long QT syndrome in the Japanese population. *Journal of Human Genetics,* 2000, vol. 45, 182-183
- **OKADA J ; YOSHINAGA T ; KUROKAWA J ; WASHIO T ; FURUKAWA T ; SAWADA K ; SUGIURA S ; HISADA T.** Screening SYSTEM for drug-induced arrhythmogenic risk combining a patch clamp and heart simulator. *Science Advances.,* 2015, vol. 1 (4